(19) Europäisches Patentamt
European Patent Office
Office européen des brevets

(11) **EP 2 258 252 A1**

(12) **EUROPEAN PATENT APPLICATION**

(43) Date of publication:
**08.12.2010 Bulletin 2010/49**

(51) Int Cl.:
*A61B 1/05* (2006.01)  *G02B 23/24* (2006.01)
*A61B 1/04* (2006.01)  *A61B 1/045* (2006.01)
*A61B 1/06* (2006.01)

(21) Application number: **10164733.7**

(22) Date of filing: **02.06.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **04.06.2009 JP 2009134751**

(71) Applicant: **FUJIFILM Corporation
Minato-ku
Tokyo (JP)**

(72) Inventors:
• **Kubo, Masahiro**
  **Kanagawa-ken (JP)**
• **Okoyama, Kazuo**
  **Kanagawa-ken (JP)**
• **Kinjo, Naoto**
  **Kanagawa-ken (JP)**
• **Ohta, Yasunori**
  **Kanagawa-ken (JP)**

(74) Representative: **Klunker . Schmitt-Nilson . Hirsch
Patentanwälte
Destouchesstrasse 68
80796 München (DE)**

(54) **Endoscopic image processing apparatus, method and program**

(57)     Endoscopic image (P) of a subject is obtained by imaging the subjected illuminated with white light output from a light source unit (10). Further, the lightness and/or imaging magnification during imaging of the endoscopic image (P) is detected. A wavelength set for the spectral estimation image (SP) and matrix parameter (M) are automatically selected based on the lightness and/or the imaging magnification. Further, matrix operation is performed on the endoscopic image (P) by using the matrix parameter (M). Accordingly, the spectral estimation image (SP) is generated.

# FIG.1

EP 2 258 252 A1

**Description**

BACKGROUND OF THE INVENTION

Field of the Invention

The present invention relates to an endoscopic image processing apparatus and method for generating a spectral estimation images by performing matrix operation on an endoscopic image. Further, the present invention relates to an endoscopic image processing program.

Description of the Related Art

Endoscopic apparatuses are used to diagnose the conditions of the body cavities of patients, such as esophagi, stomachs, and large intestines, based on endoscopic images. The endoscopic apparatuses obtain the images of the body cavities of patients by inserting scopes into the body cavities. In recent years, when an endoscope is used in diagnosis, endoscopic images obtained by a scope are displayed on a monitor in real time. Therefore, a doctor checks imaged positions or regions by looking at the monitor, and diagnoses the patient based on the images (image diagnosis). Further, observation by endoscopes includes an ordinary observation mode, a fluorescent observation mode, a narrow-band mode, and the like to facilitate image diagnosis by doctors. The ordinary observation mode observes the subject illuminated with white light. The fluorescent observation mode observes fluorescence output from the subject when the subject is illuminated with fluorescence. The narrow-band mode observes the subject illuminated with narrow band light.

Further, generation of spectral images by operation processing based on image signals obtained by using white light has been proposed. The white light is used instead of narrow band light, which is used in a narrow band mode. In this method, relationships between numerical data representing the color sensitivity characteristic of each of R, G and B and numerical data representing the spectral characteristic of a specific narrow band-pass filter are obtained as matrix data (coefficient sets). Further, a spectral estimation image is obtained by estimating a spectral image obtained through the narrow band-pass filter by performing operation using the matrix data and RGB signals. When the spectral image is generated by performing operation as described above, it is not necessary to prepare a plurality of filters corresponding to desired wavelength bands. Further, it is not necessary to change or arrange the plurality of filters. Therefore, it is possible to prevent the size of the endoscopic apparatus from becoming large, and to lower the cost of the endoscopic apparatus.

Meanwhile, automatic switching of the various observation modes has been proposed so that doctors can perform efficient image diagnosis (for example, please refer to Japanese Unexamined Patent Publication No. 2007-020728 (Patent Document 1)). Patent Document 1 discloses automatic switching of various imaging modes, such as the ordinary observation mode, fluorescent observation mode and narrow-band mode, based on the magnification of a lens.

Further, automatic switching of observation mode based on the lightness of an endoscopic image instead of the magnification of the lens has been proposed (for example, please refer to Japanese Unexamined Patent Publication No. 2006-341078 (Patent Document 2)). Patent Document 2 discloses forced switching to ordinary image observation mode when the lightness of a spectral image signal becomes a value less than or equal to a predetermined threshold value.

In Patent Documents 1 and 2, the various observation modes are automatically switched based on the lightness or the magnification of the lens. However, when a spectral estimation image is generated based on an endoscopic image, it is desirable that an optimum spectral estimation image for diagnosis is automatically generated and displayed in addition to switching of the various observation modes.

SUMMARY OF THE INVENTION

In view of the foregoing circumstances, it is an object of the present invention to provide an endoscopic image processing apparatus and method that can automatically set a wavelength set and matrix parameter when a spectral estimation image is generated. Further, the present invention relates to an endoscopic image processing program.

An endoscopic image processing apparatus of the present invention is an endoscopic image processing apparatus comprising:

an image obtainment means that obtains an endoscopic image of a subject when the subject illuminated with light output from a light source unit is imaged by using a scope;
an imaging condition detection means that detects lightness (or brightness) and/or imagining magnification during imaging of the endoscopic image by the image obtainment means;
a wavelength set table that stores a plurality of wavelength sets, each including a plurality of wavelength bands;

a wavelength set selection means that selects, based on the lightness and/or imaging magnification detected by the imaging condition detection means, a wavelength set to be used to generate a spectral estimation image from the wavelength set table; and

a spectral image generation means that generates the spectral estimation image by performing matrix operation on the endoscopic image by using matrix parameter corresponding to the wavelength set selected by the wavelength set selection means.

An endoscopic image processing method of the present invention is an endoscopic image processing method comprising the steps of:

obtaining an endoscopic image of a subject when the subject illuminated with light output from a light source unit is imaged by using a scope;

detecting lightness and/or imaging magnification during imaging of the endoscopic image;

selecting, based on the detected lightness and/or imaging magnification, a wavelength set that is used to generate a spectral estimation image from a wavelength set table that stores, as each of a plurality of wavelength sets, a plurality of wavelength bands; and

generating the spectral estimation image by performing matrix operation on the endoscopic image by using matrix parameter corresponding to the selected wavelength set.

An endoscopic image processing program of the present invention causes a computer to execute the procedures of;

obtaining an endoscopic image of a subject when the subject illuminated with light output from a light source unit is imaged by using a scope;

detecting lightness and/or imaging magnification during imaging of the endoscopic image;

selecting, based on the detected lightness and/or imaging magnification, a wavelength set that is used to generate a spectral estimation image from a wavelength set table that stores, as each of a plurality of wavelength sets, a plurality of wavelength bands; and

generating the spectral estimation image by performing matrix operation on the endoscopic image by using matrix parameter corresponding to the selected wavelength set.

Here, the method adopted by the imaging condition detection means is not limited as long as the lightness during imaging (obtainment) of the endoscopic image is detected. For example, the lightness (or brightness) of the endoscopic image may be detected. Alternatively, when the light source unit includes a diaphragm for automatically adjusting the amount of light illuminating a subject so that the lightness of the endoscopic image becomes a predetermined value, the lightness may be detected based on the aperture value of the diaphragm.

Further, the imaging condition detection means should detect the imaging magnification during imaging of the endoscopic image. For example, the imaging condition detection means may detect an optical imaging magnification in a scope. Alternatively, the imaging condition detection means may detect an imaging magnification by an electronic zoom.

Further, when the scope has a retractable hood (projectable/retractable hood) at the leading end thereof, the imaging condition detection means may have a function for detecting whether the hood is in a non-retracted state (projected state) or not. When the imaging condition detection means detects that the hood is in a non-retracted state, the wavelength set selection means may select a wavelength set corresponding to a case in which the lightness is the highest and/or the imaging magnification is the highest.

The wavelength set table may store not only the wavelength sets but a plurality of gain coefficients for respective RGB components of the spectral estimation image based on the lightness. In such a case, the wavelength set selection means may select, from the wavelength set table, the gain coefficients corresponding to the lightness as well as the wavelength set. Further, the spectral image generation means may generate the spectral estimation image by using the wavelength set and the gain coefficients.

According to the endoscopic image processing apparatus, method, and program of the present invention, an endoscopic image of a subject is obtained when the subject illuminated with light output from a light source unit is imaged by using a scope. Further, lightness and/or imaging magnification during imaging of the endoscopic image is detected. Further, the wavelength set that is used to generate a spectral estimation image is selected, based on the detected lightness and/or imaging magnification, from a wavelength set table that stores, as each of a plurality of wavelength sets, a plurality of wavelength bands. Further, the spectral estimation image is generated by performing matrix operation on the endoscopic image by using matrix parameter corresponding to the selected wavelength set. Therefore, it is possible to estimate a region that a user wants to observe based on the lightness and/or the magnification and to automatically set a wavelength set that is appropriate for the region. Hence, it is possible to improve the efficiency of diagnosis.

When the light source unit has a diaphragm for automatically adjusting the amount of light illuminating the subject so that the lightness of the endoscopic image becomes a predetermined value, and the imaging condition detection means detects the lightness based on the aperture value of the diaphragm, it is possible to accurately detect the lightness under AEC control, in which the amount of illumination light is automatically controlled.

Further, when the scope includes a retractable hood at the leading end thereof, and the imaging condition detection means detects that the lightness is the highest and/or the imaging magnification is the highest when the retractable hood is in a non-retracted state (projected state), it is possible to judge that close-up imaging (short-distance view) is performed if the hood is used, and to estimate that the lightness is the highest and the imaging magnification is high. Accordingly, efficient automatic selection of the wavelength set is possible.

Further, when the wavelength set table stores, based on the lightness, a plurality of gain coefficients for respective RGB components of the spectral estimation image, and the wavelength set selection means selects, from the wavelength set table, the gain coefficients corresponding to the lightness as well as the wavelength set, and the spectral image generation means generates the spectral estimation image by using the wavelength set and the gain coefficients, it is possible to obtain appropriate RGB component values based on the lightness. Hence, it is possible to improve the image quality of the spectral image.

Note that the program of the present invention may be provided being recorded on a computer readable medium. Those who are skilled in the art would know that computer readable media are not limited to any specific type of device, and include, but are not limited to: floppy disks, CD's, RAM's, ROM's, hard disks, magnetic tapes, and internet downloads, in which computer instructions can be stored and/or transmitted. Transmission of the computer instructions through a network or through wireless transmission means is also within the scope of this invention. Additionally, computer instructions include, but are not limited to: source, object and executable code, and can be in any language including higher level languages, assembly language, and machine language.

BRIEF DESCRIPTION OF THE DRAWINGS

Figure 1 is a block diagram illustrating an endoscopic apparatus using an endoscopic image processing apparatus according to an embodiment of the present invention;

Figure 2 is a table showing an example of matrix parameter stored in a database in the endoscopic image processing apparatus illustrated in Figure 1;

Figure 3 is a table showing an example of a plurality of wavelength sets stored in a wavelength set table illustrated in Figure 1;

Figure 4 is a table showing corresponding relationships of lightness and imaging magnification with wavelength sets; and

Figure 5 is a flow chart illustrating an endoscopic image processing method according to an embodiment of the present invention.

DESCRIPTION OF THE PREFERRED EMBODIMENTS

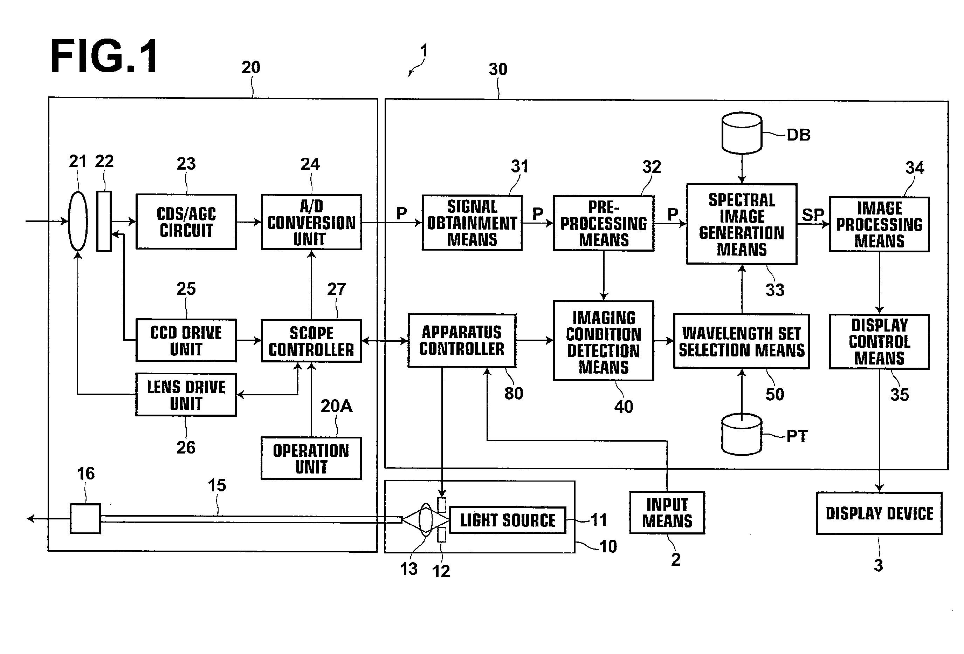

Hereinafter, embodiments of the present invention will be described in detail with reference to drawings. Figure 1 is a block diagram illustrating an example of an endoscopic apparatus 1 to which an endoscopic image processing apparatus of the present invention has been applied. The endoscopic apparatus 1 includes a light source unit 10, a scope 20, and an endoscopic image processing apparatus 30. The light source unit 10 outputs light to a subject to observe the subject by an endoscope. The light source unit 10 includes a light source 11, a diaphragm 12, and a condensing lens (condenser) 13. The light source 11 is a xenon lamp or the like, which outputs light to perform ordinary observation. The diaphragm 12 is AEC (auto exposure control) controlled by an apparatus controller 80. When white light is output from the light source 11, the white light enters a light guide 15 through the diaphragm 12 and the condensing lens 13. Further, the white light is output from an observation window 16 to the subject.

The scope 20 includes an imaging lens 21, an imaging device 22, a CDS/AGC circuit 23, an A/D converter 24, a CCD drive unit 25, a lens drive unit 26, and the like. Each element of the scope 20 is controlled by a scope controller 27. The imaging lens 21 is composed of a plurality of lens groups for example. The imaging magnification of the imaging lens 21 is changed by being driven by the lens drive unit 26. The imaging device 22 includes, for example, a CCD, a CMOS, or the like. The imaging device 22 obtains an image by performing photoelectric conversion on an image of the subject formed by the imaging lens 21. As the imaging device 22, a complementary color type device, or a primary color type device is used for example. The complementary color type device has a color filter of Mg (magenta), Ye (yellow), Cy (cyan), and G (green) on the imaging surface thereof. The primary color type device has a color filter of RGB. Further, the operation of the imaging device 22 is controlled by the CCD drive unit 25. When the imaging device 22 obtains image (video) signals, the CDS/AGC (correlated double sampling / automatic gain control) circuit 23 performs sampling on the image (video) signals, and amplifies the sampled signals. Further, the A/D converter 24 performs A/D conversion on an endoscopic image output from the CDS/AGC circuit 23, and outputs the converted endoscopic image to the endoscopic image processing apparatus 30.

The endoscopic image processing apparatus 30 processes the endoscopic image P, obtained by the scope 20. For example, the endoscopic image processing apparatus 30 is configured by a DSP (digital signal processor) or the like. The endoscopic image processing apparatus 30 includes an image obtainment means 31, a pre-processing means 32, a spectral image generation means 33, an image processing means 34, and a display control means 35. The image obtainment means 31 obtains the endoscopic image P imaged by the imaging device 22 in the scope 20. The pre-processing means 32 performs pre-processing on the endoscopic image P obtained by the image obtainment means 31. For example, when the endoscopic image P is represented in a YCC color system, the pre-processing means 32 converts the endoscopic image P into an image represented in an RGB color system. Further, the pre-processing means 32 has a gamma conversion function (gamma correction function), a gradation adjustment function, and the like.

The spectral image generation means 33 generates spectral estimation image SP by performing matrix operation on the endoscopic image P by using matrix parameter M. An example of the operation by the spectral image generation means 33 is described in detail in Japanese Unexamined Patent Publication No. 2003-093336.

Specifically, the spectral image generation means 33 generates the spectral estimation image SP by performing matrix operation using the following formula (1):

[Formula 1]

$$\begin{pmatrix} SP_r \\ SP_g \\ SP_b \end{pmatrix} = \begin{pmatrix} M_{00} & M_{01} & M_{02} \\ M_{10} & M_{11} & M_{12} \\ M_{20} & M_{21} & M_{22} \end{pmatrix} \cdot \begin{pmatrix} Pr \\ Pg \\ Pb \end{pmatrix} \qquad \cdots (1)$$

In Formula (1), $Sp_r$, $Sp_g$, $Sp_b$ represent R, G and B components of the spectral estimation image SP, respectively. Pr, Pg and Pb represent R, G and B components of the endoscopic image P, respectively. Values $M_{00}$ through $M_{22}$ in the matrix of 3 x 3 (three columns and three rows) represent matrix parameters M for matrix operation.

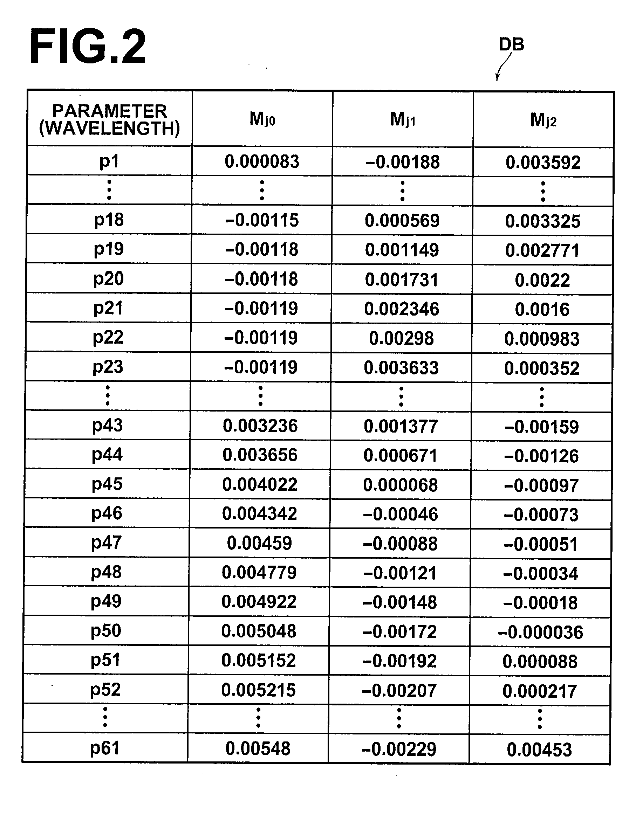

Figure 2 is a table showing an example of database DB that stores matrix parameter for performing matrix operation represented by Formula (1). In Figure 2, the database DB stores parameter pi = ($M_{j0}$, $M_{j1}$, $M_{j2}$) (the sign "i" is used to distinguish parameter sets stored in the database DB from each other, and i = 1 through 61, and "j" represents the rows of matrix M in Formula (1), and j = 0 through 2). The parameter pi is stored for each of 61 wavelength bands, into which the wavelength band of 400 nm to 700 nm is divided at 5 nm intervals for example.

The image processing means 34 performs enhancement processing or the like on spectral estimation image SP. The display control means 35 has a function for displaying the endoscopic image P on which image processing has been performed by the image processing means 34. The display control means 35 displays the endoscopic image P on a display device 3 together with character information or the like.

Here, the endoscopic image processing apparatus 30 has a function for automatically selecting a wavelength set based on an imaging condition when the aforementioned spectral estimation image SP is generated. The endoscopic image processing apparatus 30 includes an imaging condition detection means 40 and a wavelength set selection means 50. The imaging condition detection means 40 detects lightness during imaging (obtainment) of endoscopic image P by the scope 20. Specifically, the imaging condition detection means 40 detects the lightness during imaging based on the aperture value of the diaphragm 12 that is under AEC control. Here, the imaging condition detection means 40 detects the lightness based on the aperture value of the diaphragm 12. However, when the aforementioned AEC control is not performed, the lightness during imaging may be detected by performing histogram analysis or the like on the endoscopic image P.

Further, the imaging condition detection means 40 has a function for detecting the imaging magnification during imaging of an endoscopic image. The imaging condition detection means 40 detects an optical imaging magnification in the scope 20 through the apparatus controller 80, or an imaging magnification by an electronic zoom.

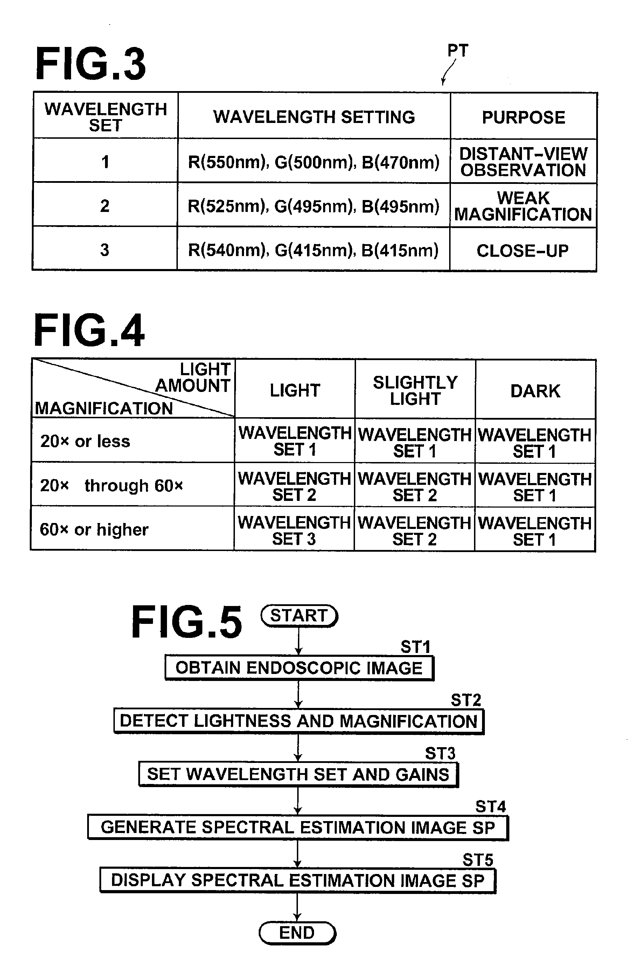

The imaging condition detection means 40 may have a function for detecting whether a hood attached to the leading end of the scope 20 is projected or housed (non-retracted or retracted). When the hood projects from the leading end of the scope 20 (or the hood is in a non-retracted state), the imaging condition detection means 40 may judge that the imaging condition is close-up imaging (short distance imaging) in which the leading end of the scope 20 is close to the subject. Therefore, the imaging condition detection means 40 may judge that the lightness is the highest and the imaging magnification is the highest, and select wavelength set 3 from wavelength sets 1 through 3 in Figure 4.

The wavelength set selection means 50 has a function for selecting the wavelength set of the spectral estimation image SP based on the lightness and the imaging magnification detected by the imaging condition detection means 40. Specifically, as illustrated in Figure 3, wavelength set table PT, in which a plurality of wavelength sets 1 through 3 are stored, is prepared in advance. The wavelength set selection means 50 automatically selects a wavelength set from the wavelength sets 1 through 3 stored in the wavelength set table PT. For example, the wavelength set table PT illustrated in Figure 3 stores wavelength set 1 (R component = 550 nm, G component = 500 nm, and B component = 470 nm), wavelength set 2 (R component = 525 nm, G component = 495 nm, B component = 495 nm), and wavelength set 3 (R component = 540 nm, G component = 415 nm, and B component = 425 nm).

The wavelength set selection means 50 includes a table showing relationships of lightness and imaging magnification with wavelength sets 1 through 3. In Figure 4, the lightness and the imaging magnification are classified into three categories, and in combination, into nine types. The wavelength set selection means 50 selects a wavelength set from the wavelength sets 1 through 3 based on the lightness / imaging magnification. Further, the spectral image generation means 33 selects matrix parameter M corresponding to the selected wavelength set from database DB, and performs matrix operation based on Formula (1). Accordingly, spectral estimation image SP is generated.

As stated above, the wavelength set is automatically selected based on the lightness and the imaging magnification. Therefore, it is possible to automatically select a wavelength set appropriate for the imaging condition and the intension of a user to generate the spectral estimation image SP. Hence, the user does not need to manually select a wavelength set, and the efficiency of diagnosis is improved. Specifically, in a conventional method, although a plurality of wavelength sets are prepared in advance, the user needs to manually select a wavelength from the plurality of wavelength sets based on a region to be observed. Therefore, a longer time period or work is required to display the spectral estimation image SP. However, in the present invention, the region to be observed (the region that the user wants to observe) can be estimated based on the lightness and the imaging magnification during imaging of the endoscopic image. Therefore, it is possible to save the time and work of selecting the wavelength set by the user by automatically selecting a wavelength set from the wavelength sets 1 through 3. The wavelength set is automatically selected based on the lightness and the imaging magnification to generate the spectral estimation image SP. Hence, efficient image diagnosis is possible.

Further, the endoscopic image processing apparatus 30 may have a function for adjusting the gain for each of RGB components based on the lightness. Specifically, the spectral image generation means 33 generates spectral estimation image SP by using matrix parameter M1, as represented in the following Formula (1'), instead of the matrix parameter M. The matrix parameter M1 is obtained by multiplying the matrix parameter M by gain coefficients Rg, Gg and Bg of respective RGB components.

[Formula 1']

$$M1 = \begin{pmatrix} Rg & 0 & 0 \\ 0 & Gg & 0 \\ 0 & 0 & Bg \end{pmatrix} \cdot \begin{pmatrix} M_{00} & M_{01} & M_{02} \\ M_{10} & M_{11} & M_{12} \\ M_{20} & M_{21} & M_{22} \end{pmatrix} \quad \cdots \quad (1')$$

The gain coefficients Rg, Gg, and Bg are changed based the lightness during imaging of an endoscopic image. For example, in Figure 4, when the imaging magnification is less than or equal to 20x (20-power, or 20 times), wavelength set 1 is selected without regard to the category into which the lightness is classified. However, different gain coefficient Rg, Gg and Bg are stored for each of the categories of the lightness. Specifically, even if the wavelength set selection means 50 selects the wavelength set 1, different gain coefficients Rg, Gg and Bg are selected based on the lightness. Since the gain coefficients Rg, Gg, and Bg for the respective RGB components are set based on the lightness, it is possible to improve the image quality of the spectral estimation image SP.

Figure 4 illustrates an example in which the wavelength set is switched to wavelength sets 1 through 3 based on the lightness when the imaging magnification is higher than or equal to 60x (60-power, or 60 times). Alternatively, even if the imaging magnification is higher than or equal to 60x, the same wavelength set 3 may be selected without regard to the category into which the lightness is classified, and different gain coefficients Rg, Gg, and Bg may be selected based on the category of the lightness.

Figure 5 is a flow chart illustrating an endoscopic image processing method according to an embodiment of the present invention. With reference to Figures 1 through 5, the endoscopic image processing method will be described. First, imaging is performed by inserting a scope 20 into the body cavity of a patient to obtain endoscopic image P (step ST1). Meanwhile, the imaging condition detection means 40 detects the lightness and the imaging magnification during imaging of the endoscopic image P (step ST2).

After then, the wavelength set selection means 50 selects one of the wavelength sets 1 through 3 and the gain coefficients Rg, Gg, and Bg based on the lightness and the imaging magnification (step ST3). Further, the spectral image generation means 33 generates spectral estimation image SP by using Formula (1) or (1') (step ST4) . Then, the image processing means 34 performs predetermined image processing, and the display control means 35 displays the spectral estimation image SP on the display device 3 (step ST5).

According to the embodiment of the present invention as described above, endoscopic image P of a subject illuminated with white light output from the light source unit 10 is imaged by using the scope 20. Further, the lightness and/or imaging magnification during imaging of the endoscopic image P is detected. A wavelength set to be used to generate spectral estimation image SP is selected, based on the detected lightness and/or imaging magnification, from wavelength set table PT. The wavelength table PT stores, as wavelength sets 1 through 3, a plurality of wavelengths to be represented by the spectral estimation image SP. Further, matrix operation is performed on the endoscopic image P by using matrix parameter M corresponding to the selected wavelength set to generate the spectral estimation image SP. Therefore, it is possible to estimate the region to be observed based on the lightness and/or imaging magnification, and to automatically set a wavelength set that is appropriate for observation of the region. Hence, the diagnosis efficiency is improved.

When the light source unit 10 has a diaphragm for automatically adjusting the amount of light illuminating the subject so that the lightness of the endoscopic image P becomes a predetermined value, and the imaging condition detection means 40 detects the lightness based on the aperture value of the diaphragm 12, it is possible to detect lightness under AEC control, in which the amount of illumination light is automatically controlled.

Further, when the scope 20 has a retractable hood at the leading end thereof, and the imaging condition detection means detects that the lightness is the highest and/or the imaging magnification is the highest when the hood is in a non-retracted state, it is possible to estimate that the lightness is the highest and the imaging magnification is the highest when the hood is used, because imaging using the hood is close-up imaging. Hence, automatic selection of the wavelength is performed efficiently.

Further, when the wavelength set table PT stores a plurality of gain coefficients Rg, Gg and Bg for respective RGB components of a spectral estimation image, the gain coefficients being set based on lightness, and the wavelength set selection means 50 selects a wavelength from the wavelength set table PT and the gain coefficients Rg, Gg and Bg corresponding to the lightness, and the spectral image generation means 33 generates spectral estimation image SP by using the wavelength set and the gain coefficients Rg, Gg and Bg, it is possible to obtain values of RGB components that are appropriate for the lightness. Hence, the image quality of the spectral estimation image SP is improved.

The embodiments of the present invention are not limited to the above embodiments. For example, in the above embodiments, the imaging condition detection means 40 selects the wavelength set by using both of the lightness and the imaging magnification. Alternatively, the imaging condition detection means 40 may select the wavelength set by using one of the lightness and the imaging magnification (please refer to Figure 4).

Figure 3 illustrates an example in which the wavelength set table PT stores three wavelength sets 1 through 3. However, it is not necessary that the number of the wavelength sets is three, and the number of the wavelength sets may be two or greater than three. Further, the imaging condition detection means 40 classifies each of the lightness and the imaging magnification into three categories. Alternatively, the imaging condition detection means 40 may classify the lightness and/or the imaging magnification into a plurality of cases.

An example in which the endoscopic image processing apparatus 30 is configured by hardware, such as a DSP, has been described. Alternatively, the endoscopic image processing apparatus 30 may be configured by a computer, such as a personal computer. In such a case, the configuration of the endoscopic image processing apparatus 30 illustrated in Figure 1 is realized by causing a computer (for example, a personal computer or the like) to execute an endoscopic image processing program that has been read in an auxiliary storage apparatus.

**Claims**

1.   An endoscopic image processing apparatus comprising:

an image obtainment means (31) that obtains an endoscopic image (P) of a subject when the subject illuminated with light output from a light source unit (10) is imaged by using a scope (20);
an imaging condition detection means (40) that detects lightness and/or imagining magnification during imaging of the endoscopic image (P) that has been obtained by the image obtainment means (31);
a wavelength set table (PT) that stores a plurality of wavelength sets, each including a plurality of wavelength bands;
a wavelength set selection means (50) that selects, based on the lightness and/or imaging magnification detected by the imaging condition detection means (40), a wavelength set to be used to generate a spectral estimation image (SP) from the wavelength set table (PT); and

a spectral image generation means (33) that generates the spectral estimation image (SP) by performing matrix operation on the endoscopic image (P) by using matrix parameter (M) corresponding to the wavelength set selected by the wavelength set selection means (50).

2. An endoscopic image processing apparatus, as defined in Claim 1, wherein the light source unit (10) has a diaphragm (12) for automatically adjusting the amount of light illuminating the subject so that the lightness of the endoscopic image (P) becomes a predetermined value, and wherein the imaging condition detection means (40) detects the lightness based on the aperture value of the diaphragm (12).

3. An endoscopic image processing apparatus, as defined in Claim 1 or 2, wherein the scope (20) includes a retractable hood at the leading end thereof, and wherein the imaging condition detection means (40) detects that the lightness is the highest and/or the imaging magnification is the highest when the retractable hood is in a non-retracted state.

4. An endoscopic image processing apparatus, as defined in any one of Claims 1 to 3, wherein the wavelength set table (PT) stores, based on the lightness, a plurality of gain coefficients for respective RGB components of the spectral estimation image (SP), and wherein the wavelength set selection means (50) selects, from the wavelength set table (PT), the gain coefficients (Rg, Gg, Bg) corresponding to the lightness as well as the wavelength set, and wherein the spectral image generation means (33) generates the spectral estimation image (SP) by using the wavelength set and the gain coefficients (Rg, Gg, Bg).

5. An endoscopic image processing method comprising the steps of:

obtaining an endoscopic image (P) of a subject when the subject illuminated with light output from a light source unit (10) is imaged by using a scope (20);

detecting lightness and/or imaging magnification during imaging of the endoscopic image (P);

selecting, based on the detected lightness and/or imaging magnification, a wavelength set that is used to generate a spectral estimation image (SP) from a wavelength set table (PT) that stores, as each of a plurality of wavelength sets, a plurality of wavelength bands; and

generating the spectral estimation image (SP) by performing matrix operation on the endoscopic image (P) by using matrix parameter (M) corresponding to the selected wavelength set.

6. An endoscopic image processing program for causing a computer to execute the procedures of;

obtaining an endoscopic image (P) of a subject when the subject illuminated with light output from a light source unit (10) is imaged by using a scope (20);

detecting lightness and/or imaging magnification during imaging of the endoscopic image (P);

selecting, based on the detected lightness and/or imaging magnification, a wavelength set that is used to generate a spectral estimation image (SP) from a wavelength set table (PT) that stores, as each of a plurality of wavelength sets, a plurality of wavelength bands; and

generating the spectral estimation image (SP) by performing matrix operation on the endoscopic image (P) by using matrix parameter (M) corresponding to the selected wavelength set.

# FIG.1

# FIG.2

DB

| PARAMETER (WAVELENGTH) | $M_{j0}$ | $M_{j1}$ | $M_{j2}$ |
|---|---|---|---|
| p1 | 0.000083 | −0.00188 | 0.003592 |
| ⋮ | ⋮ | ⋮ | ⋮ |
| p18 | −0.00115 | 0.000569 | 0.003325 |
| p19 | −0.00118 | 0.001149 | 0.002771 |
| p20 | −0.00118 | 0.001731 | 0.0022 |
| p21 | −0.00119 | 0.002346 | 0.0016 |
| p22 | −0.00119 | 0.00298 | 0.000983 |
| p23 | −0.00119 | 0.003633 | 0.000352 |
| ⋮ | ⋮ | ⋮ | ⋮ |
| p43 | 0.003236 | 0.001377 | −0.00159 |
| p44 | 0.003656 | 0.000671 | −0.00126 |
| p45 | 0.004022 | 0.000068 | −0.00097 |
| p46 | 0.004342 | −0.00046 | −0.00073 |
| p47 | 0.00459 | −0.00088 | −0.00051 |
| p48 | 0.004779 | −0.00121 | −0.00034 |
| p49 | 0.004922 | −0.00148 | −0.00018 |
| p50 | 0.005048 | −0.00172 | −0.000036 |
| p51 | 0.005152 | −0.00192 | 0.000088 |
| p52 | 0.005215 | −0.00207 | 0.000217 |
| ⋮ | ⋮ | ⋮ | ⋮ |
| p61 | 0.00548 | −0.00229 | 0.00453 |

# FIG.3

PT

| WAVELENGTH SET | WAVELENGTH SETTING | PURPOSE |
|---|---|---|
| 1 | R(550nm), G(500nm), B(470nm) | DISTANT-VIEW OBSERVATION |
| 2 | R(525nm), G(495nm), B(495nm) | WEAK MAGNIFICATION |
| 3 | R(540nm), G(415nm), B(415nm) | CLOSE-UP |

# FIG.4

| LIGHT AMOUNT / MAGNIFICATION | LIGHT | SLIGHTLY LIGHT | DARK |
|---|---|---|---|
| 20× or less | WAVELENGTH SET 1 | WAVELENGTH SET 1 | WAVELENGTH SET 1 |
| 20× through 60× | WAVELENGTH SET 2 | WAVELENGTH SET 2 | WAVELENGTH SET 1 |
| 60× or higher | WAVELENGTH SET 3 | WAVELENGTH SET 2 | WAVELENGTH SET 1 |

# FIG.5

START

ST1
OBTAIN ENDOSCOPIC IMAGE

ST2
DETECT LIGHTNESS AND MAGNIFICATION

ST3
SET WAVELENGTH SET AND GAINS

ST4
GENERATE SPECTRAL ESTIMATION IMAGE SP

ST5
DISPLAY SPECTRAL ESTIMATION IMAGE SP

END

Europäisches
Patentamt

European
Patent Office

Office européen
des brevets

## EUROPEAN SEARCH REPORT

Application Number

EP 10 16 4733

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 880 657 A1 (OLYMPUS MEDICAL SYSTEMS CORP [JP]) 23 January 2008 (2008-01-23) * paragraphs [0021], [0027] - [0029], [0063], [0065], [0090] - [0096], [0101] - [0102], [0133], [0137], [0197], [0201], [0289] * ----- | 1-6 | INV. A61B1/05 G02B23/24 A61B1/04 A61B1/045 A61B1/06 |
| Y | EP 1 698 272 A2 (FUJINON CORP [JP]) 6 September 2006 (2006-09-06) * paragraphs [0001], [0006] - [0022], [0037], [0041], [0054] - [0056] * * claim 3 * * figures 1, 2, 4 * ----- | 1-6 | |

TECHNICAL FIELDS
SEARCHED (IPC)

A61B
G02B

The present search report has been drawn up for all claims

| Place of search | Date of completion of the search | Examiner |
|---|---|---|
| Munich | 20 August 2010 | Denise, Christophe |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 10 16 4733

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2010

| Patent document cited in search report | | Publication date | Patent family member(s) | | Publication date |
|---|---|---|---|---|---|
| EP 1880657 | A1 | 23-01-2008 | AU | 2006245251 A1 | 16-11-2006 |
| | | | BR | PI0609099 A2 | 17-02-2010 |
| | | | CA | 2607623 A1 | 16-11-2006 |
| | | | WO | 2006120798 A1 | 16-11-2006 |
| | | | KR | 20080002948 A | 04-01-2008 |
| | | | RU | 2381737 C2 | 20-02-2010 |
| | | | US | 2009023991 A1 | 22-01-2009 |
| EP 1698272 | A2 | 06-09-2006 | US | 2006211915 A1 | 21-09-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007020728 A
- JP 2006341078 A

- JP 2003093336 A